# EUROPEAN PATENT APPLICATION

(11) **EP 3 895 656 A1**
(43) Date of publication of application: **20.10.2021**
(21) Application number: 19895720.1
(22) Date of filing: 24.07.2019
(51) Int. Cl.: A61C 17/06, A61C 17/08, A61M 1/00

(54) **SUCTION TIP**

(30) Priority: 11.12.2018 KR 20180158859
(71) Applicant: DMAX INTERNATIONAL CO., LTD., Seoul 04511 (KR)
(72) Inventor: KIM, Jaeyoun, Seoul 05507 (KR)
(74) Representative: M. Zardi & Co S.A.
(86) International application number: PCT/KR2019/009143
(87) International publication number: WO 2020/122346

(57) **Abstract**

A suction tip according to the present invention is coupled to an end of a suction tube connected to a suction device and includes a body part having an internal space into which the suction tube is inserted, and a center hole formed at a center of a front end thereof and communicating with the internal space, in which the body part includes a plurality of suction holes disposed radially around the center hole. The plurality of suction holes of the suction tip is formed to be deepened from a rear end to a front end of the body part, and the suction tip has a plurality of guide grooves so that even though a buccal mucosa or a tongue covers a circular outer surface part of the suction tip, a negative pressure for suction is easily and uninterruptedly transmitted along the guide grooves extending to the rear end of the body part, thereby maintaining a suction function.

## Description

### [Technical Field]

The present invention relates to a suction tip, and particularly, to a suction tip capable of maintaining a suction function by easily and uninterruptedly transmitting a negative pressure for suction even though soft tissue such as a buccal mucosa or a tongue is covered.

### [Background Art]

A suction device, which is mainly used in dentistry, is widely used for the purpose of suctioning an extracted object such as saliva, blood, water, tartar, and residues in a patient's oral cavity during a dental procedure.

A suction tip of the suction device has a suction port for suctioning the extracted object, and a practitioner uses the suction tip in the patient's oral cavity in order to suction the extracted object through the suction port of the suction tip.

During the use of the suction tip, soft tissue such as a buccal mucosa or a tongue in the oral cavity is sucked into the suction port, which frequently clogs the suction port.

Because a suction function is stopped when the suction port is clogged as described above, a practitioner needs to inconveniently stop a treatment and adjust a position of the suction tip again.

In particular, the practitioner performs the treatment in a state in which the suction device is hung on a part of the oral cavity when the practitioner performs the treatment such as the removal of tartar alone without an assistant's help. In this state, the suction tip is positioned almost horizontally instead of being positioned at a right angle or in an oblique line. For this reason, the soft tissue in the oral cavity is sucked into the suction port of the suction tip, and the suction port is more frequently clogged.

### [Disclosure]

### [Technical Problem]

The present invention has been made in an effort to solve the various problems in the related art, and an object of the present invention is to provide a suction tip capable of maintaining a suction function by easily and uninterruptedly transmitting a negative pressure for suction even though soft tissue such as a buccal mucosa or a tongue is covered.

### [Technical Solution]

In order to achieve the above-mentioned object, the present invention provides a suction tip coupled to an end of a suction tube connected to a suction device, the suction tip including: a body part having an internal space into which the suction tube is inserted and having a center hole formed at a center at a front end thereof and communicating with the internal space, in which the body part includes a plurality of suction holes disposed radially around the center hole and formed spirally toward the center hole.

In this case, the plurality of suction holes may be formed to be deepened from a rear end to a front end of the body part.

In addition, the plurality of suction holes may be formed spirally toward the center hole of the body part.

In addition, the body part may further include a plurality of guide grooves spaced apart from the plurality of suction holes in a direction of a center axis and formed at predetermined intervals in a circumferential direction.

In addition, the plurality of guide grooves may extend to a rear end of the body part.

In addition, the plurality of guide grooves may be formed spirally toward the center hole of the body part.

In addition, a through hole may be formed at one end of each of the plurality of guide grooves and communicates with the internal space of the body part.

The through hole may extend in a direction different from a longitudinal direction of the plurality of guide grooves.

In addition, the body part may further include a first protrusion and a second protrusion formed in an inner surface thereof, the first protrusion may be formed at a rear end with respect to the through hole, and the second protrusion may be formed between the through holes and the plurality of suction holes.

In addition, the end of the suction tube may be coupled to the inner surface of the body part in an interference fit manner.

### [Advantageous Effects]

According to the present invention, the plurality of suction holes of the suction tip is formed to be deepened from a rear end to a front end of the body part, and the suction tip has a plurality of guide grooves so that even though a buccal mucosa or a tongue covers a circular outer surface part of the suction tip, a negative pressure for suction is easily and uninterruptedly transmitted along the guide grooves extending to the rear end of the body part, thereby maintaining a suction function.

In addition, according to the suction tip according to the present invention, since the plurality of suction holes and the plurality of guide grooves are spirally formed toward the center hole of the body part, the surface areas of the suction holes and the guide grooves may be increased, such that it is possible to increase the areas that cannot be clogged by the extracted objects and the soft tissue such as the buccal mucosa or the tongue.

In addition, according to the suction tip according to the present invention, since the through hole is formed at one end of each of the plurality of guide grooves and extends in the direction different from the longitudinal direction of the guide groove, it is possible to significantly reduce a likelihood that the inlet into which the extracted objects are sucked is clogged.

### [Description of Drawings]

FIGS. 1A and 1B are a perspective top plan view and a perspective bottom plan view of a suction tip according to an embodiment of the present invention.
FIG. 2 is a top plan view of the suction tip according to the embodiment of the present invention.
FIG. 3 is a view schematically illustrating a cross section of the suction tip according to the embodiment of the present invention.
FIG. 4 is a cross-sectional view schematically illustrating a state in which a suction tube is inserted into and coupled to a position at which a first protrusion of a body part is formed in the suction tip according to another embodiment of the present invention.
FIG. 5 is a cross-sectional view schematically illustrating a state in which the suction tube is inserted into and coupled to a position at which a second protrusion of the body part is formed in the suction tip according to another embodiment of the present invention.

### [Mode for Invention]

Hereinafter, embodiments of the present invention will be described in detail with reference to the accompanying drawings. First, in assigning reference numerals to constituent elements of the respective drawings, it should be noted that the same constituent elements will be designated by the same reference numerals, if possible, even though the constituent elements are illustrated in different drawings. In addition, in the description of the present invention, the descriptions of configurations will be omitted when it is determined that the descriptions may obscure the subject matter of the present invention. Further, the embodiments of the present invention will be described below, but the technical spirit of the present invention is not limited thereto and may of course be carried out by those skilled in the art.

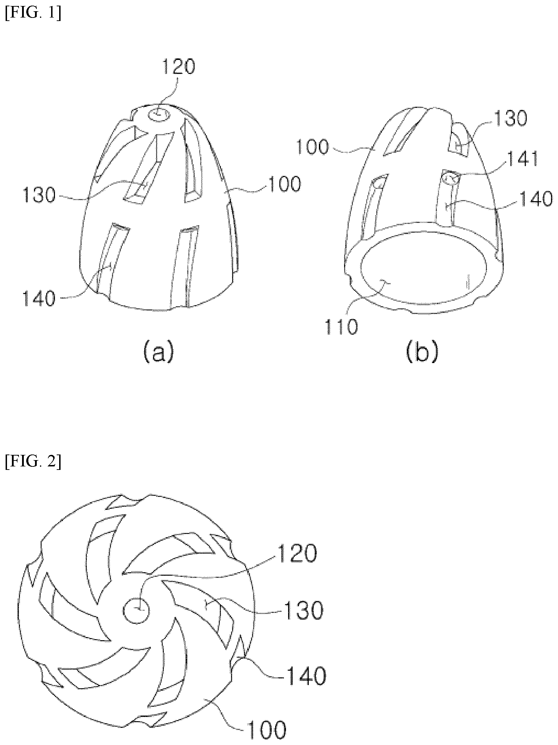

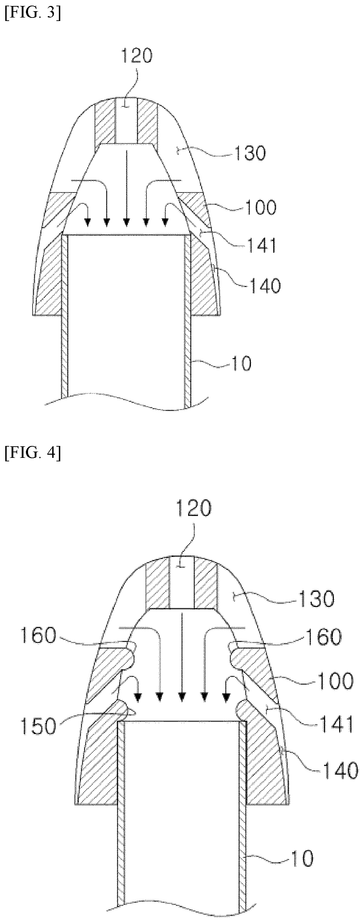

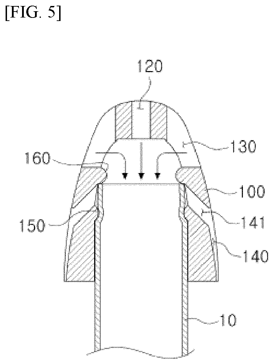

FIGS. 1A and 1B are a perspective top plan view and a perspective bottom plan view of a suction tip according to an embodiment of the present invention, FIG. 2 is a top plan view of the suction tip according to the embodiment of the present invention, FIG. 3 is a view schematically illustrating a cross section of the suction tip according to the embodiment of the present invention, FIG. 4 is a cross-sectional view schematically illustrating a state in which a suction tube is inserted into and coupled to a position at which a first protrusion of a body part is formed in the suction tip according to another embodiment of the present invention, and FIG. 5 is a cross-sectional view schematically illustrating a state in which the suction tube is inserted into and coupled to a position at which a second protrusion of the body part is formed in the suction tip according to another embodiment of the present invention.

Hereinafter, a suction tip according to the embodiment of the present invention will be described with reference to FIGS. 1 to 3.

Referring to FIGS. 1 to 3, the suction tip according to the present invention is configured such that an end of a suction tube 10 connected to a suction device (not illustrated) is inserted into and coupled to an internal space 110 of a body part 100, and a practitioner may separate the coupled end of the suction tube 10 from the body part 100 by pulling the suction tube 10 outward.

In this case, the suction device is connected to a suction motor (not illustrated), and extracted objects such as patient's saliva, blood, water, tartar, and residues may be sucked into the suction tube 10 and received in a separately provided receiving portion (not illustrated) by a negative pressure provided by the suction motor.

In addition, a wire (not illustrated) is provided in the suction tube 10, such that the practitioner may bend the suction tube 10 in a desired shape.

Meanwhile, the suction tip according to the present invention may be made of, but not limited to, plastic, rubber, silicone, synthetic resin, or the like.

The suction tip includes the body part 100. The body part 100 may have a circular cross section, and an outer portion at a front end of the body part 100 may be formed in a hemispheric shape or a cylindrical shape.

In addition, the body part 100 has the internal space 110 into which the suction tube 10 is inserted. That is, the practitioner may couple the body part 100 and the suction tube 10 by pushing the body part 100 and the suction tube 10 in a direction in which the body part 100 and the suction tube 10 move toward each other.

In addition, a center hole 120 communicating with the internal space 110 is formed at a center of the front end of the body part 100.

When the negative pressure is transmitted through the suction tube 10 in a state in which the body part 100 and the suction tube 10 are coupled to each other as described above, the extracted objects such as saliva, blood, water, tartar, and residues made during a dental treatment may be sucked into the suction tube 10 through the center hole 120 of the body part 100.

Meanwhile, the body part 100 has a plurality of suction holes 130 communicating with the internal space 110. The plurality of suction holes 130 may be disposed radially around the center hole 120 formed in the body part 100. That is, the plurality of suction holes 130 may be formed in a peripheral region of the center hole 120 and disposed at predetermined intervals.

In addition, the plurality of suction holes 130 is formed to be deepened from a rear end toward a front end of the body part 100. That is, since a portion adjacent to the center hole 120 of the body part 100 is relatively deeper, the negative pressure for suction may be easily and uninterruptedly transmitted even though a buccal mucosa or a tongue covers some of the plurality of suction holes 130.

In addition, the plurality of suction holes 130 is formed spirally toward the center hole 120 of the body part 100. Since the plurality of suction holes 130 is formed spirally as described above, surface areas of the suction holes 130 are increased, such that areas, which cannot be clogged by the soft tissue such as the buccal mucosa or the tongue and the extracted objects, are increased, and as a result, it is possible to reduce a likelihood that the suction hole 130 is clogged.

In addition, a spiral vortex of the extracted objects is induced as the extracted objects are sucked along the plurality of suction holes 130 formed spirally, and the extracted objects may be sucked into the internal space 110 of the body part 100 while rotating and swirling. That is, the extracted objects may be quickly sucked while being rotated by the spiral vortex.

Meanwhile, a plurality of guide grooves 140 is formed to be spaced apart from the plurality of suction holes 130 in a direction of a center axis and disposed at predetermined intervals in a circumferential direction.

In this case, the plurality of guide grooves 140 extends to the rear end of the body part 100 and is formed spirally toward the center hole 120 of the body part 100.

In addition, a through hole 141 is formed at one end of each of the plurality of guide grooves 140 and communicates with the internal space 110 of the body part 100. In this case, the through hole 141 may extend in a direction different from a longitudinal direction of each of the plurality of guide grooves 140.

Since the through hole 141 is formed at one end of each of the plurality of guide grooves 140 as described above, a suction function may be maintained because the extracted objects may be sucked through the through hole 141, which extends in the direction different from the longitudinal direction of the guide groove 140 from one end of the guide groove 140 and communicates with the internal space 110 of the body part 100, even though the soft tissue such as the buccal mucosa or the tongue is sucked into the guide groove 140 and the guide groove 140 is clogged.

That is, because the suction hole 130 formed in the suction tip in the related art is formed in one direction which is the direction in which the extracted objects are sucked, an inlet into which the extracted objects are sucked is clogged immediately when the suction hole 130 is clogged by the soft tissue. In contrast, according to the present invention, since the through hole 141 is formed at one end of each of the plurality of guide grooves 140 and provided in the direction different from the direction of the guide groove 140, the inlet into which the extracted objects are sucked is clogged only when the plurality of guide grooves 140 and the through holes 141 are clogged at the same time.

Therefore, in comparison with the related art, the suction tip according to the present invention may significantly reduce a likelihood that the inlet into which the extracted objects are sucked is clogged.

Hereinafter, a suction tip according to another embodiment of the present invention will be described with reference to FIGS. 4 and 5.

Unlike the suction tip illustrated in FIGS. 1 to 3, the suction tip according to another embodiment illustrated in FIGS. 4 and 5 includes a first protrusion 150 and a second protrusion 160 formed on an inner surface of the body part.

The first protrusion 150 may be formed at the rear end with respect to the through hole 141. In addition, the second protrusion 160 may be formed between the through holes 141 and the plurality of suction holes 130.

When the practitioner performs the treatment, such as the removal of tartar, alone without an assistant's help, the practitioner may insert the suction tube 10 to a position at which the first protrusion 150 of the body part 100 is formed, thereby coupling the body part 100 and the suction tube 10 in an interference fit manner, as illustrated in FIG. 4.

That is, the practitioner may easily couple the body part 100 and the suction tube 10 with his/her hand without using an adhesive means such as a bonding agent. In addition, when the suction tube 10 is inserted to the first protrusion 150 of the body part 100, the plurality of through holes 141 communicates with the internal space 110 of the body part 100. Therefore, even though the guide groove 140 is clogged, the extracted objects may be sucked through the through hole 141 extending in the direction different from the longitudinal direction of the guide groove 140, such that the suction function may be maintained.

Meanwhile, in a situation in which an assistant may help the practitioner, the practitioner may insert the suction tube 10 to a position at which the second protrusion 160 of the body part 100 is formed, thereby coupling the body part 100 and the suction tube 10 in an interference fit manner, as illustrated in FIG. 5.

In this case, even though the plurality of through holes 141 is clogged by the suction tube 10, the negative pressure may be concentrated in the center hole 120 and the plurality of suction holes 130 during the suction.

Therefore, in a situation in which the assistant may assist in adjusting the position of the suction tip when the plurality of suction holes 130 is clogged by the soft tissue or the extracted objects, the practitioner may insert the suction tube 10 to the position illustrated in FIG. 5 in an interference fit manner and perform the suction, thereby concentrating the negative pressure in the center hole 120 and the plurality of suction holes 130.

Meanwhile, as illustrated in FIGS. 4 and 5, the first protrusion 150 and the second protrusion 160 may be formed in, but not limited to, a hemispheric shape, but the shape and the number of the first and second protrusions 150 and 160 may be changed by those skilled in the art, as necessary.

While the exemplary embodiments of the present invention have been described in detail above, the technical scope of the present invention is not limited to the above-mentioned embodiments and should be defined by the appended claims. In this case, it will be interpreted by those skilled in the art that various modifications and alterations may be made without departing from the scope of the present disclosure.

## Claims

1. A suction tip coupled to an end of a suction tube connected to a suction device, the suction tip comprising:
a body part having an internal space into which the suction tube is inserted and having a center hole formed at a center at a front end thereof and communicating with the internal space,
wherein the body part comprises a plurality of suction holes disposed radially around the center hole and formed spirally toward the center hole.

2. The suction tip of claim 1, wherein the plurality of suction holes is formed to be deepened from a rear end to a front end of the body part.

3. The suction tip of claim 1, wherein the body part further comprises a plurality of guide grooves spaced apart from the plurality of suction holes in a direction of a center axis and formed at predetermined intervals in a circumferential direction.

4. The suction tip of claim 3, wherein the plurality of guide grooves extends to a rear end of the body part.

5. The suction tip of claim 4, wherein the plurality of guide grooves is formed spirally toward the center hole of the body part.

6. The suction tip of claim 5, wherein a through hole is formed at one end of each of the plurality of guide grooves and communicates with the internal space of the body part.

7. The suction tip of claim 6, wherein the through hole extends in a direction different from a longitudinal direction of the plurality of guide grooves.

8. The suction tip of claim 7, wherein the body part further comprises a first protrusion and a second protrusion formed in an inner surface thereof, the first protrusion is formed at a rear end with respect to the through hole, and the second protrusion is formed between the through holes and the plurality of suction holes.

9. The suction tip of claim 8, wherein the end of the suction tube is coupled to the inner surface of the body part in an interference fit manner.
